# EUROPEAN PATENT APPLICATION

(11) **EP 4 548 960 A1**
(43) Date of publication of application: **07.05.2025**
(21) Application number: 24208648.6
(22) Date of filing: 24.10.2024
(51) Int. Cl.: A61N 1/32, A61N 1/04, A61N 1/36

(54) **ELECTRICAL MUSCLE STIMULATION SYSTEM AND ELECTRICAL MUSCLE STIMULATION METHOD CAPABLE OF IMPLEMENTING A ROLLING MASSAGE BENEFIT THROUGH INDIVIDUAL CHANNEL CONTROL**

(30) Priority: 31.10.2023 KR 20230148445
(71) Applicant: Coremovement Co., Ltd., Busan 48548 (KR)
(72) Inventor: KIM, Myeongcheol, 48299 Busan (KR); PARK, Jun Sul, 48228 Busan (KR); AN, Chan Hyeok, 47592 Busan (KR)
(74) Representative: Schmitt-Nilson Schraud Waibel Wohlfrom Patentanwälte Partnerschaft mbB

(57) **Abstract**

Disclosed are an electrical muscle stimulation system and an electrical muscle stimulation method which implement a rolling massage benefit that provides a massage while moving along a user's body parts. An electrical muscle stimulation system includes a main body provided to be wearable by a user, or provided in a bathtub-like form or in a form capable of coming into contact with a user's body, a plurality of electrode pads provided on the main body and spaced apart from each other at positions corresponding to various parts of the user's body, and a control unit that individually controls operations of the plurality of electrode pads by outputting control signals to the plurality of electrode pads through individual channels. The control unit may sequentially operate the plurality of electrode pads according to an arrangement order of the plurality of electrode pads to implement a rolling massage mode.

## Description

### CROSS-REFERENCE TO RELATED APPLICATIONS

This application claims priority under 35 U.S.C. § 119 to Korean Patent Application No. 10-2023-0148445 filed on October 31, 2023, in the Korean Intellectual Property Office, the disclosures of which are incorporated by reference herein in their entireties.

### BACKGROUND

Embodiments of the present disclosure described herein relate to an electrical muscle stimulation system, and more particularly, relate to an electrical muscle stimulation system and an electrical muscle stimulation method which implement a rolling massage that provides a massage while moving along a user's body parts.

Recently, an electrical muscle stimulation apparatus has been utilized to enhance the benefits of massage, exercise, or treatment, for example. The electrical muscle stimulation (EMS) apparatus may electrically stimulate muscles adjacent to a position where an EMS electrode is attached to contract the muscles, thereby allowing the user to obtain benefits such as increased muscle strength, blood circulation promotion, fatigue recovery, and massage without separate exercise or strain on joints due to the separate exercise.

Conventional electrical muscle stimulation apparatuses have limitations in that a control signal is transmitted to multiple electrode pads through a single channel, making it difficult to focus electrical stimulation on a specific body part desired by the user. In addition, since electrical muscle stimulation is applied uniformly through the electrode pads, there is a limitation in that it is difficult to achieve rolling massage benefit that provides sequential/continuous massage while moving along the user's body parts.

The present disclosure provides an electrical muscle stimulation system and an electrical muscle stimulation method capable of implementing a rolling massage benefit that provides a massage while moving along body parts of a user by sequentially applying an electrical stimulation signal to a plurality of electrode pads through individual channel control.

In addition, the present disclosure provides an electrical muscle stimulation system and an electrical muscle stimulation method capable of providing a massage continuously/sequentially along body parts of a user by controlling an electrical stimulation pattern applied through a plurality of electrode pads.

In addition, the present disclosure provides an electrical muscle stimulation system and an electrical muscle stimulation method capable of providing a water receiving portion in an electrode pad to effectively induce an electrical stimulation to a user through water received in a water receiving portion.

In addition, the present disclosure provides an electrical muscle stimulation system and an electrical muscle stimulation method capable of controlling the reinforcement/cancellation interference of electrical signals between adjacent electrode pads such that an appropriate electrical stimulation required by a user is induced.

The problems to be solved by the present disclosure are not limited to the problems mentioned above. Other technical problems not mentioned will be clearly understood by those of ordinary skill in the art to which the present disclosure pertains from the following description.

### SUMMARY

According to an embodiment, an electrical muscle stimulation system includes a main body provided to be wearable by a user, or provided in a bathtub-like form or in a form capable of coming into contact with a user's body, a plurality of electrode pads provided on the main body and spaced apart from each other at positions corresponding to various parts of the user's body, and a control unit that individually controls operations of the plurality of electrode pads by outputting control signals to the plurality of electrode pads through individual channels. The control unit may sequentially apply an electrical stimulation signal according to an arrangement order of the plurality of electrode pads to implement a rolling massage mode.

The control unit may sequentially apply an electric stimulation signal to the plurality of electrode pads to operate the plurality of electrode pads. The electrical stimulation signal may include a stimulation increase interval in which an electrical stimulation gradually increases, a stimulation maintenance interval in which the electrical stimulation is maintained, and a stimulation decrease interval in which the electrical stimulation gradually decreases.

Electrical stimulation signals generated from adjacent pairs of electrode pads include an overlapping stimulation interval that partially overlaps each other. The overlapping stimulation interval, electrical stimulation signals generated from one pair of electrode pads may gradually decrease, and electrical stimulation signals generated from the other pair of electrode pads may gradually increase.

The overlapping stimulation interval may include a resonance prevention interval in which electrical stimulation signals generated from two pairs of electrode pads are set such that the a current generated from the one pair of electrode pads and a current generated from the other pair of electrode pads do not cause resonance, and a resonance induction interval in which the electrical stimulation signals generated from the two pairs of electrode pads are set such that the current generated from the one pair of electrode pads and the current generated from the other pair of electrode pads cause resonance.

The electrical muscle stimulation system may further include a water receiving portion provided in at least one of the main body and the electrode pad to receive water between the user's body and the electrode pad.

The water receiving portion may include a water receiving groove including a water receiving space provided in the main body to receive water, and the electrode pad may include a recess electrode installed in a buried manner within the water receiving groove.

The water receiving portion may include a water receiving member provided on the electrode pad or a water receiving groove formed by patterning an upper surface of the electrode pad.

According to an embodiment, an electrical muscle stimulation method includes individually controlling, by a control unit, operations of a plurality of electrode pads provided in a main body by outputting control signals to the plurality of electrode pads through individual channels, the main body being provided to be wearable by a user, or provided in a bathtub-like form or in a form capable of coming into contact with a user's body, and the plurality of electrode pads being provided on the main body and spaced apart from each other at positions corresponding to various parts of the user's body, wherein the controlling includes sequentially applying an electrical stimulation signal according to an arrangement order of the plurality of electrode pads to implement a rolling massage mode.

The implementing of the rolling massage mode may include sequentially applying, by the control unit, an electrical stimulation signal to the plurality of electrode pads to operate the plurality of electrode pads. The electrical stimulation signal may include a stimulation increase interval in which electrical stimulation gradually increases, a stimulation maintenance interval in which electrical stimulation is maintained, and a stimulation decrease interval in which electrical stimulation gradually decreases.

According to an embodiment of the present disclosure, a computer program recorded on a computer-readable, non-transitory recording medium is provided to execute the electrical muscle stimulation method.

### BRIEF DESCRIPTION OF THE FIGURES

The above and other objects and features will become apparent from the following description with reference to the following figures, wherein like reference numerals refer to like parts throughout the various figures unless otherwise specified, and wherein:
FIG. 1 is a diagram showing an electrical muscle stimulation system according to an embodiment of the present disclosure;
FIG. 2 is a conceptual diagram showing a state of use of the electrical muscle stimulation system according to the embodiment of FIG. 1;
FIG. 3 is a diagram showing a view from above of a bathtub constituting the electrical muscle stimulation system of FIG. 1;
FIG. 4 is a diagram showing an electrical muscle stimulation system according to another embodiment of the present disclosure;
FIG. 5 is a configuration diagram of an electrical muscle stimulation system according to an embodiment of the present disclosure;
FIG. 6 is a conceptual diagram showing the implementation of rolling massage benefit for a user by an electrical muscle stimulation system according to an embodiment of the present disclosure;
FIGS. 7 to 9 are example diagrams showing electrical stimulation signals applied through individual channels to a plurality of electrode pads constituting an electrical muscle stimulation system to implement rolling massage benefit for a user according to an embodiment of the present disclosure; and
FIGS. 10 to 13 are enlarged views of portion 'A' of FIG. 6, showing electrode pads and a water receiving portion constituting an electrical muscle stimulation system according to an embodiment of the present disclosure.

### DETAILED DESCRIPTION

Advantages and features of the present disclosure and methods for achieving them will be apparent with reference to embodiments described below in detail in conjunction with the accompanying drawings. However, the present disclosure is not limited to the embodiments disclosed below, but may be implemented in various different forms. The present embodiments are provided only to make the disclosure of the present disclosure complete and to fully inform those skilled in the art of the invention of the scope of the invention, and the present disclosure is defined only by the scope of the claims.

Unless defined herein, all terms (including technical or scientific terms) used herein have the same meaning as commonly accepted by general technology in the prior art to which the present disclosure belongs. Terms defined by general dictionaries are to be interpreted as having the same meaning as they have in the relevant art and/or the text of this application.

Like reference numerals refer to like elements throughout the specification. The present disclosure does not describe all elements of the embodiments, and general content in the technical field to which the present disclosure pertains or overlapping content between embodiments are omitted. As used herein, '^{~}unit' is a unit for processing at least one function or operation, and may refer to, for example, software, FPGA, or hardware component.

In this specification, the function provided by ''unit' may be performed separately by a plurality of components, or may be integrated with other additional components. A plurality of "units" may be implemented as a single component. The term '^{~}unit' in this specification is not necessarily limited to software or hardware, and may be configured to reside in an addressable storage medium, or may be configured to reproduce one or more processors.

Terms such as first and second are used to distinguish one component from another component, and the components are not limited by the above-mentioned terms. As used herein, singular forms may include plural forms as well unless the context clearly indicates otherwise. As used herein, the term 'and/or' refers to each of listed components or various combinations thereof. Hereinafter, the operating principle and embodiments of the present disclosure will be described with reference to the attached drawings.

An electrical muscle stimulation system and an electrical muscle stimulation method according to an embodiment of the present disclosure are provided to provide an electrical muscle stimulation system and an electrical muscle stimulation method capable of implementing rolling massage benefit that provides a massage by sequentially/continuously moving along a body part such as a user's back, neck, waist, thigh, or calf by sequentially generating electrical stimulation signals through a plurality of electrode pads of the electrical muscle stimulation system.

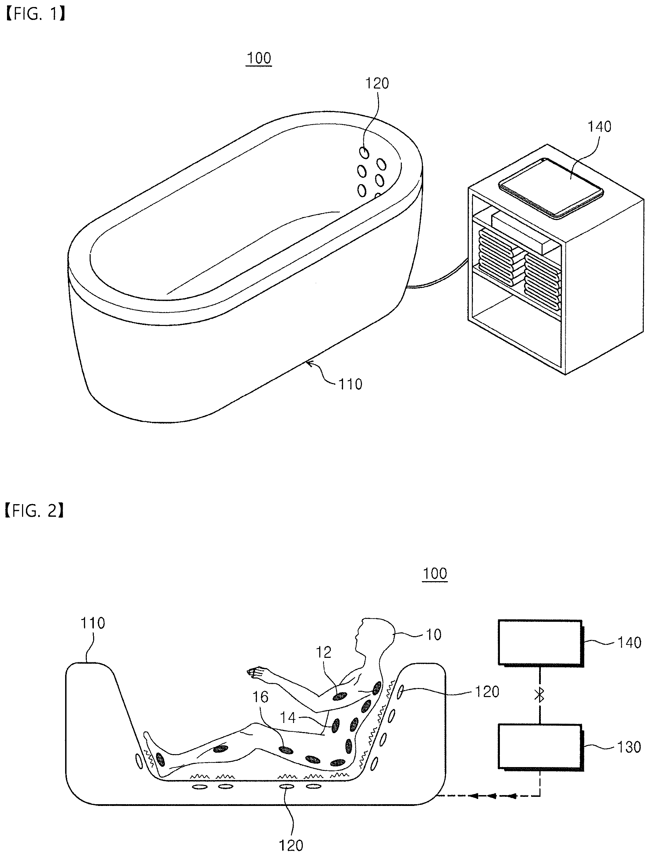

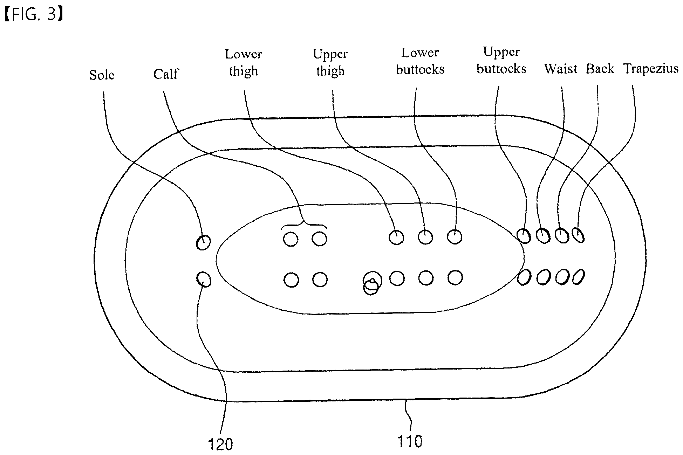

FIG. 1 is a diagram showing an electrical muscle stimulation system according to an embodiment of the present disclosure. FIG. 2 is a conceptual diagram showing a state of use of the electrical muscle stimulation system according to the embodiment of FIG. 1. FIG. 3 is a diagram showing a view from above of a bathtub constituting the electrical muscle stimulation system of FIG. 1.

Referring to FIGS. 1 to 3, an electrical muscle stimulation system 100 according to an embodiment of the present disclosure may include a main body 110 implemented in the form of a bathtub or the like, a plurality of electrode pads 120 provided in a distributed manner on the main body 110, a control device 130 configured to control the plurality of electrode pads 120 to generate an electrical signal for electrical muscle stimulation through at least one operating electrode pad among the plurality of electrode pads 120, and a user terminal 140.

The electrical muscle stimulation system 100 may be used by a user in a bathtub in the form of a spa, or may be used in the air or underwater in the form of a suit, band, patch, or the like. The user may receive health care, rehabilitation treatment, or massage in such a way that muscles are electrically stimulated by the electrical muscle stimulation system 100 while being located in a bathtub equipped with the plurality of electrode pads 120, or wearing a suit, band, patch, or the like including the electrical muscle stimulation system 100 in the air or underwater.

The main body 110 is a component that supports the plurality of electrode pads 120, and may be provided in the form of a bathtub as shown in FIG. 1 such that the user is able to receive electrical muscle stimulation in the form of a spa, may be provided in the form of a suit, band, or patch capable of being worn by the user, or may be provided to come into contact with the user's body In addition, the main body 110 may be provided as a silicone pad (EMS pad with multiple electrode pads attached) that may be attached to a bathtub without electrode pads and used.

The plurality of electrode pads 120 may be provided on one or more main bodies 110 and spaced apart from each other at positions corresponding to various parts of the user's body. The electrode pads 120 are made of a conductive material and may be placed on the inner surface of the main body 110 adjacent to the user's body part (for example, the inner surface of the bathtub) so as to effectively transmit electrical stimulation to the user's body part, such as the user's muscles.

Each of the electrode pads 120 may selectively operate as an operating electrode pad according to a control signal transmitted from the control device 130 via an individual channel. The electrode pad 120 operating as an operating electrode pad may generate an electrical stimulation signal for applying electrical stimulation to the user's body part, such as the user's muscles, according to the control signal received from the control device 130.

The control device 130 may be provided in the main body 110 or may be provided outside the main body 110. The control device 130 may be connected to each electrode pad 120 through wired or wireless communication. The control device 130 may include a control unit. The control unit may include at least one processor and at least one memory. The control unit may output control signals through a plurality of individual channels to the plurality of electrode pads 120. The control unit may individually control the plurality of electrode pads 120 according to the control signals transmitted through the plurality of individual channels.

For example, the control signal output from the control unit may be an electrical stimulation signal. That is, an electrical signal output unit (not shown) is provided in the control unit, and the control signal may be transmitted to the electrode pad 120 in the form of an electrical stimulation signal through the individual channels and output through the electrode pad 120. In this case, the control signal may be transmitted to the electrode pad 120 in the form of an electrical pulse having a set pulse size and a set pulse frequency.

For another example, the control signal output from the control unit may be transmitted to the electrode pad 120 as an electrode control signal rather than an electrical pulse. In this case, the control signal output from the control unit may include pulse size information and pulse frequency information of an electrical stimulation signal to be output through the electrode pad 120, or may include information for identifying the electrical pulse of an electrical stimulation signal, and the electrode pad 120 may be equipped with an electrical signal output unit that generates and outputs an electrical pulse according to the control signal received from the control unit.

The control device 130 may be equipped with an input unit (not shown) that allows a user to input a massage mode for electrical muscle stimulation. When a massage mode for electrical muscle stimulation to be induced on the user's body is input by the input unit of the control device 130, the control unit may select one or more operating electrode pads among the plurality of electrode pads 120 according to the input massage mode for electrical muscle stimulation, and determine a control signal to be applied to the selected operating electrode pad.

In an embodiment, the user may input a massage mode by inputting one or more body parts 12, 14, or 16 among various body parts such as shoulders, chest, neck, arms, wrists, waists, thighs, and calves, a massage intensity, a massage duration, or the like through the input unit. In another example, the user may input the massage mode for electrical muscle stimulation by touching a position where the electrical muscle stimulation is to be applied or focused on a human body image displayed on the input unit.

The control unit of the control device 130 may sequentially select one or more operating electrode pads matching the massage mode (or rolling mode) for electrical muscle stimulation input by the user through the input unit from among the plurality of electrode pads 120. To this end, the position information of each electrode pad 120 provided on the main body 110 or the identification information of the electrode pad 120 may be set in advance in the storage unit of the control device 130.

The electrode pad 120 may generate an electrical signal according to the control signal transmitted from the control unit of the control device 130 and provide an electrical stimulus to the user's muscles corresponding to the massage mode for electrical muscle stimulation. The current intensity and/or frequency of the electrical signal generated by the electrode pad 120 may be adjusted according to the control signal transmitted from the control unit through an individual channel.

The control device 130 may individually (independently) set a level (current/voltage intensity level and/or frequency level) of the electrical signal generated for each part through the electrode pad 120. The control device 130 may further include a notification unit (not shown). The notification unit may perform a function of notifying a user of various electrical muscle stimulation information such as the position and intensity of an electrical stimulus applied to the user, or the massage/rolling mode. Any method such as a method for displaying information on a display screen or a method for outputting sound through a speaker may be used as long as the notification unit notifies the user of information.

The bathtub corresponding to the main body 110 may be replaced with various products that may be installed in various facilities such as homes, accommodation facilities, and businesses to receive water and for people to enter. The electrode pad 120 may be provided in an area of the inner area of the main body 110 in the form of a bathtub, capable of coming into contact with the human body. The electrode pad 120 may output an electrical pulse according to a control signal in the form of an electrical signal provided by the control device 130 to apply an electrical stimulus to a human body, thereby stimulating the user's motor nerves or providing a massage benefit. Through this, the user may strengthen the muscles of the body or obtain a massage benefit.

For example, the electrode pad 120 may be provided in an area capable of contacting or being adjacent to at least one of the trapezius, back, waist, upper buttocks, lower buttocks, upper thighs, lower thighs, calves, or soles when a person lies in a bathtub. The bathtub may include a built-in battery for operating the electrode pads 120 and the control device 130, and may be connected to the control device 130 or an external power source via a wire.

The control unit of the control device 130 may include an oscillation unit. The oscillation unit of the control device 130 may generate an electrical pulse through the electrode pad 120 based on control data, and may generate an electrical pulse having various sizes and frequencies depending on the purpose, such as massage, muscle strengthening, and electrical stimulation. For example, the oscillation unit may include a pulse generation unit that generates an electrical pulse, a display unit that displays a pulse output mode or pulse output intensity, and an adjustment unit that adjusts the pulse output mode or pulse output intensity. The control device 130 may generate control data for controlling an electrical pulse, for example, data for controlling the magnitude of a current or voltage (pulse size) constituting the pulse or the level of the pulse, such as frequency (pulse frequency).

The oscillation unit may generate an electrical pulse whose magnitude and frequency are controlled based on the control data generated by the control device 130, and the generated electrical pulse may be output through the electrode pad 120. The control device 130 may receive, from the user, a control command to control the magnitude of the electrical pulse or the level of the electrical pulse, such as frequency and then generate control data based on the control command. The control data generated as described above may be used to control the electrical pulse generated by the oscillation unit.

The user terminal 140 may be provided as a device such as a smart phone, tablet, or PC. A storage medium in the user terminal 140 may store an application capable of controlling the electrical muscle stimulation system 100. The user of the electrical muscle stimulation system 100 may run the application installed in the user terminal 140 and then input a control command. The user terminal 140 may generate control data based on the input control command and transmit the control data to the control device 130 via wired/wireless communication. The electrical pulse generated through the oscillation unit of the control device 130 may be controlled based on the control data transmitted from the user terminal 140.

The control command may be a command to output electrical pulses respectively having different magnitudes and frequencies for the electrode pads 120 corresponding to the target body parts 12, 14, and 16 for various electrical muscle stimulations. The control command may be a command to cause the electrode pad 120 corresponding to a thigh position to output an electrical pulse of a first level, and the electrode pad 120 corresponding to a waist position to output an electrical pulse of a second level.

The control data may be data to cause the oscillation unit of the control device 130 to generate an electrical pulse of the same level as the control command. More specifically, the first control data may be data to cause the electrode pad 120 corresponding to the thigh position to output the electrical pulse of the first level, and the electrode pad 120 corresponding to the waist position to output the electrical pulse of the second level, like the control command.

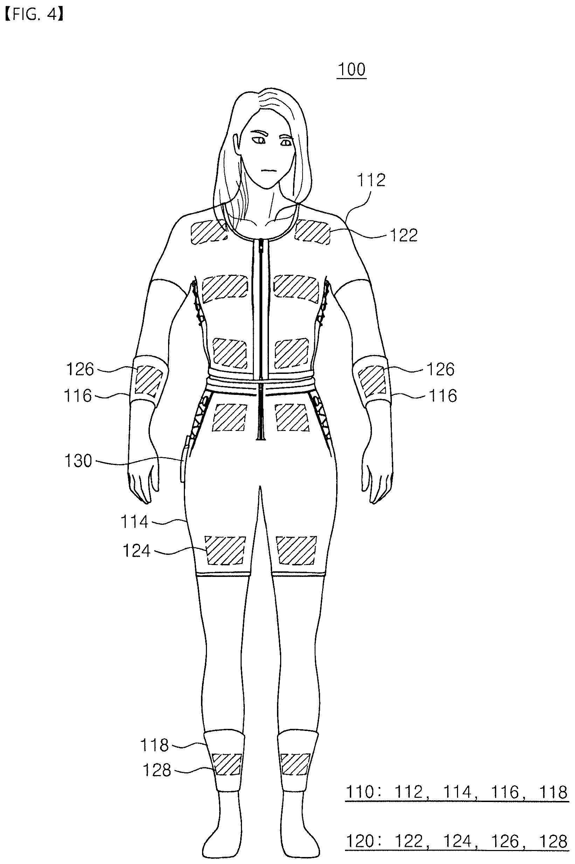

FIG. 4 is a drawing showing an electrical muscle stimulation system according to another embodiment of the present disclosure. In the embodiment of FIG. 4, the main body 110 may include an upper suit 112 provided for a user to wear on her or his upper body, a lower suit 114 provided for the user to wear on her or his lower body, and one or more bands 126 and 128 provided for the user to wear by wrapping them around a body part such as an arm, a leg, or an abdomen. Alternatively, the main body 110 may be provided in the form of a patch attached to a user's body. For example, the electrode pads 120 may include one or more electrode pads 122 provided on the upper suit 112, one or more electrode pads 124 provided on the lower suit 114, and one or more electrode pads 126 and 128 provided on one or more bands 126, 128 worn on an arm, a leg, or the like.

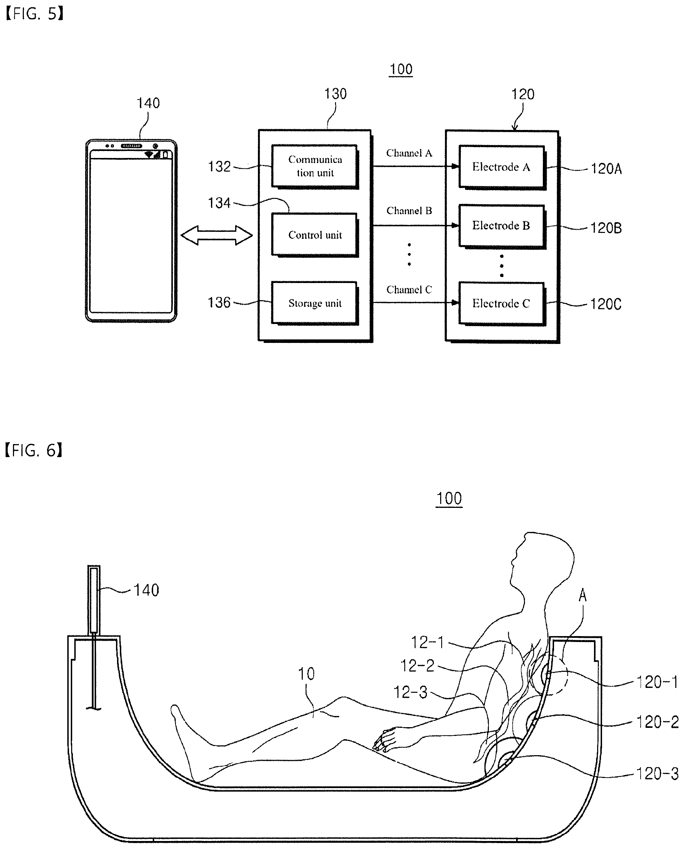

FIG. 5 is a configuration diagram of an electrical muscle stimulation system according to an embodiment of the present disclosure. Referring to FIG. 5, the plurality of electrode pads 120 (120A, 120B, and 120C) may selectively act as operating electrode pads based on control signals transmitted via individual channels (channel A, channel B, and channel C). According to a control signal provided from the control device 130 via the individual channels (Channel A, Channel B, Channel C), an operating electrode pads among the plurality of electrode pads 120 may generate an electrical stimulation signal for electrical muscle stimulation.

The control device 130 may include a communication unit 132 for communicating with the user terminal 140 and the electrode pads 120 wiredly and/or wirelessly, a control unit 134 for generating a control signal to be provided to each electrode pad 120 and outputting the control signal to each electrode pad 120 via the individual channels, and a storage unit 136 for storing various data related to electrical muscle stimulation and massage/rolling modes, and/or the like. The control unit 134 may generate a wired control signal and/or a wireless control signal for controlling the operation of the electrical muscle stimulation system 100. The communication unit 132 provided in the control device 130 may communicate with the user terminal 140 via a wired/wireless communication network, and transmit a wired/wireless control signal to the electrode pads 120 provided in a suit or bathtub, or the like.

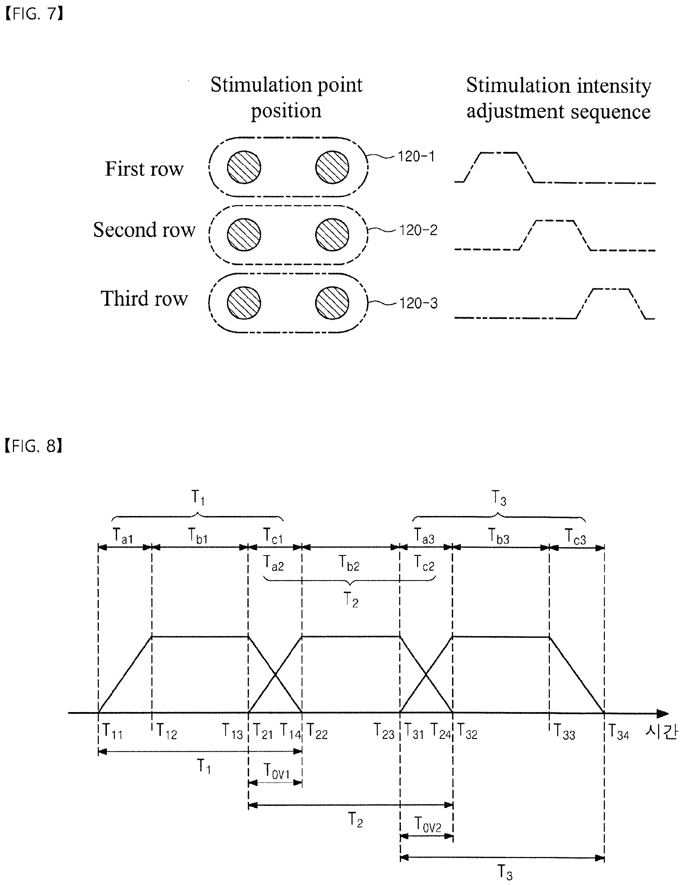

FIG. 6 is a conceptual diagram showing the implementation of rolling massage benefit for a user by an electrical muscle stimulation system according to an embodiment of the present disclosure. FIGS. 7 to 9 are example diagrams showing electrical stimulation signals applied through individual channels to a plurality of electrode pads constituting an electrical muscle stimulation system to implement rolling massage benefit for a user according to an embodiment of the present disclosure.

Referring to FIGS. 6 to 9, the control unit of the electrical muscle stimulation system 100 may sequentially apply electrical stimulation signals to a plurality of pairs of electrode pads 120-1, 120-2, and 120-3 for rolling massage according to the arrangement order (arrangement order according to positions of body parts to which the rolling massage is to applied). For example, the electrical muscle stimulation system 100 may sequentially generate electrical stimulation signals according to the electrode pads 120-1, 120-2, and 120-3, along the user's back from the top to the bottom, or from the bottom to the top, thereby implementing a rolling massage benefit.

In order for the user to experience the continuous rolling massage benefit, the electrical muscle stimulation system 100 may sequentially increase the intensity of electrical stimuluses to be generated by the pairs of electrode pads 120-1, 120-2, and 120-3 and then sequentially decrease the intensity after maintaining the intensity for a certain interval of time. In addition, in order to implement a continuous rolling massage benefit, the electrical stimulation signal applied to the adjacent two pairs of electrode pads 120-1 and 120-2, or 120-2 and 120-3 may include overlapping stimulation intervals Tₒᵥ₁ and Tₒᵥ₂, and in the overlapping stimulation intervals Tₒᵥ₁ and Tₒᵥ₂, the electrical stimulation signal generated from one pair of electrode pads 120-1 and 120-2 among the adjacent two pairs of electrode pads 120-1 and 120-2, or 120-2 and 120-3 may gradually decrease, and the electrical stimulation signal generated from the other pair of electrode pads 120-2 and 120-3 may gradually increase.

Each pair of electrode pads 120-1, 120-2, or 120-3 may include two electrode pads 120-1a and 120-1b or 120-2a and 120-2b, and an electrical stimulation signal may be generated by a pulse current/voltage applied between the two electrode pads forming the pair. The electrode pads 120-1, 120-2, and 120-3 may be provided in at least three pairs. In an embodiment, based on the arrangement order of three pairs of electrode pads 120-1, 120-2, and 120-3, an electrical stimulation signal may be sequentially applied to the first electrode pad 120-1, the second electrode pad 120-2, and the third electrode pad 120-3.

The first electrode pad 120-1 may generate a first electrical stimulation signal during a first stimulation interval T₁ from a first stimulation start time T₁₁ to a first stimulation end time T₁₄. The second electrode pad 120-2 may generate a second electrical stimulation signal during a second stimulation interval T₂ from a second stimulation start time T₂₁ to a second stimulation end time T₂₄. The third electrode pad 120-3 may generate a third electrical stimulation signal during a third stimulation interval T₃ from a third stimulation start time T₃₁ to a third stimulation end time T₃₄. Each electrical stimulation signal may include a stimulation increase interval Tₐ₁, Tₐ₂ or Tₐ₃, a stimulation maintenance interval T_{b1}, T_{b2}, or T_{b3}, and a stimulation decrease interval T_{c1}, T_{c2}, or T_{c3} between the stimulation start time T₁₁, T₂₁, or T₃₁ and the stimulation end time T₁₄, T₂₄, or T₃₄.

That is, each electrical stimulation signal may include the stimulation increase interval Tₐ₁, Tₐ₂, or Tₐ₃ in which the stimulation intensity gradually increases at a constant rate from the stimulation start time T₁₁, T₂₁, or T₃₁ to the stimulation increase end time T₁₂, T₂₂, or T₃₂, the stimulation maintenance interval T_{b1}, T_{b2}, or T_{b3} in which the stimulation intensity is maintained from a stimulation increase end time T₁₂, T₂₂, or T₃₂ to a stimulation decrease start time T₁₃, T₂₃, or T₃₃, and the stimulation decrease interval T_{c1}, T_{c2}, or T_{c3} in which the stimulation intensity gradually decreases at a constant rate from the stimulation decrease start time T₁₃, T₂₃, or T₃₃ to the stimulation end time T₁₄, T₂₄, or T₃₄.

The first electrical stimulation signal generated by the first electrode pad 120-1 may continue until after the second electrical stimulation signal has started to be generated by the second electrode pad 120-2. The second electrical stimulation signal generated by the second electrode pad 120-2 may start while the first electrical stimulation signal is being generated by the first electrode pad 120-1 and may continue until after the first electrical stimulation signal has ended. The third electrical stimulation signal generated by the third electrode pad 120-3 may start while the second electrical stimulation signal is being generated by the second electrode pad 120-2 and may continue until after the second electrical stimulation signal has ended.

During the first overlapping stimulation interval Tₒᵥ₁ between the first electrical stimulation signal of the first electrode pad 120-1 and the second electrical stimulation signal of the second electrode pad 120-2, that is, from the second stimulation start time T₂₁ of the second electrode pad 120-2 to the first stimulation end time T₁₄ of the first electrode pad 120-1, the first electrical stimulation signal of the first electrode pad 120-1 may gradually decrease, and the second electrical stimulation signal of the second electrode pad 120-2 may gradually increase.

During the second overlapping stimulation interval Tₒᵥ₂ between the second electrical stimulation signal of the second electrode pad 120-2 and the third electrical stimulation signal of the third electrode pad 120-3, that is, from the third stimulation start time T₃₁ of the third electrode pad 120-3 to the second stimulation end time T₂₄ of the second electrode pad 120-2, the second electrical stimulation signal of the second electrode pad 120-2 may gradually decrease, and the third electrical stimulation signal of the third electrode pad 120-3 may gradually increase.

In this way, during the overlapping stimulation interval Tₒᵥ₁ or Tₒᵥ₂ between two adjacent pairs of electrode pads 120-1 and 120-2, or 120-2 and 120-3, as the electrical stimulation signal of one pair of electrode pads 120-1 and 120-2 may gradually decrease and at the same time the electrical stimulation signal of the other pair of electrode pads 120-2 and 120-3 may gradually increase to cause the user to experience the stimulation moving continuously/gradually along a specific body part 12-1, 12-2, or 12-3, thus implementing a rolling massage benefit.

The time or stimulation increase/decrease speed (slope), number of repetitions and cycle of stimulation of the stimulation increase interval Tₐ₁, Tₐ₂, or Tₐ₃, stimulation maintenance interval T_{b1}, T_{b2}, or T_{b3}, stimulation decrease interval T_{c1}, T_{c2}, or T_{c3}, and overlapping stimulation interval Tₒᵥ₁ or Tₒᵥ₂ may be preset, but may also be adjusted according to a rolling massage mode set by the user, or may be adjusted in various ways according to the user's gender, age, or purpose of use (exercise, physical recovery, massage, etc.).

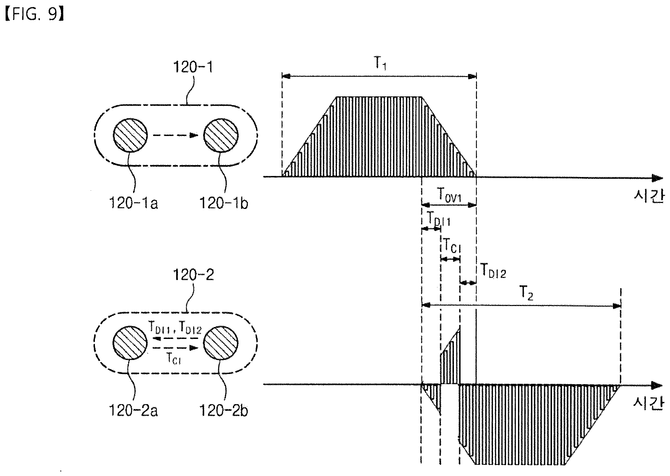

In the embodiment illustrated in FIG. 9, the overlapping stimulation interval Tₒᵥ₁ between adjacent pairs of electrode pads 120-1 and 120-2 may include a resonance prevention interval T_{DI1} or T_{DI2} and a resonance induction interval T_{CI}. The resonance prevention interval T_{DI1} or T_{DI2} may be an interval in which excessive stimulation is prevented from occurring due to the resonance phenomenon of electrical stimulation signals between the first electrode pad 120-1 and the second electrode pad 120-2, and the resonance induction interval T_{CI} may be an interval in which the resonance phenomenon of electrical stimulation signals is induced between the first electrode pad 120-1 and the second electrode pad 120-2 to strengthen stimulation in the middle region between the first electrode pad 120-1 and the second electrode pad 120-2.

During the overlapping stimulation interval Tₒᵥ₁, the resonance phenomenon of the electrical stimulation signals between the first electrode pad 120-1 and the second electrode pad 120-2 is prevented in the first resonance prevention interval T_{DI1} to allow a user not to feel a strong stimulation in a specific body part, while allowing the user to gradually feel a decrease in the first electrical stimulation signal at the first body part 12-1 adjacent to the first electrode pad 120-1.

In the resonance induction interval T_{CI} following the first resonance prevention interval T_{DI1}, a resonance phenomenon of electrical stimulation signals is induced between the first electrode pad 120-1 and the second electrode pad 120-2, thereby allowing the user to feel increasingly stronger stimulation in the middle region between the first electrode pad 120-1 and the second electrode pad 120-2. Accordingly, the user may feel the stimulation being gradually transmitted from the first body part 12-1 adjacent to the first electrode pad 120-1 to the second body part 12-2 adjacent to the second electrode pad 120-2.

In the second resonance prevention interval T_{DI2} following the resonance induction interval T_{CI}, the resonance phenomenon of electrical stimulation signals between the first electrode pad 120-1 and the second electrode pad 120-2 may be prevented to allow the user not to feel a strong stimulation to a specific body part, while allowing the user to gradually feel an increase in the second electrical stimulation signal at the second body part 12-2 adjacent to the second electrode pad 120-2.

The method for preventing resonance between the electrical stimulation signals of the adjacent electrode pads may be performed by setting the current directions of the electrical stimulation signals applied to the electrode pads to be opposite to each other (for example, when the direction of a current generated by one pair of electrode pads is a first direction, setting the direction of a current generated by the other pair of electrode pads to a second direction opposite to the first direction), or by setting the stimulation intensities (strength), phases, and/or frequencies of the electrical stimulation signals (electrical pulses) applied to the electrode pads to be different.

In contrast, the method for inducing resonance between the electrical stimulation signals of the adjacent electrode pads may be performed by setting the current directions of the electrical stimulation signals applied to the electrode pads to be the same direction (for example, when the direction of a current generated by one pair of electrode pads is a first direction, setting the direction of a current generated by the other pair of electrode pads to the same direction or the first direction), or by setting the stimulation intensities (strength), phases, and/or frequencies of the electrical stimulation signals (electrical pulses) applied to the electrode pads to be identical.

In the embodiment of FIG. 9, the intensities of electrical stimulation pulses are shown with respect to the directions of currents, and in the resonance prevention intervals T_{DI1} and T_{DI2}, currents are allowed to flow in opposite directions via the first electrode pad 120-1 and the second electrode pad 120-2, and in the resonance induction interval TCI, currents are allowed to flow in the same direction via the first electrode pad 120-1 and the second electrode pad 120-2.

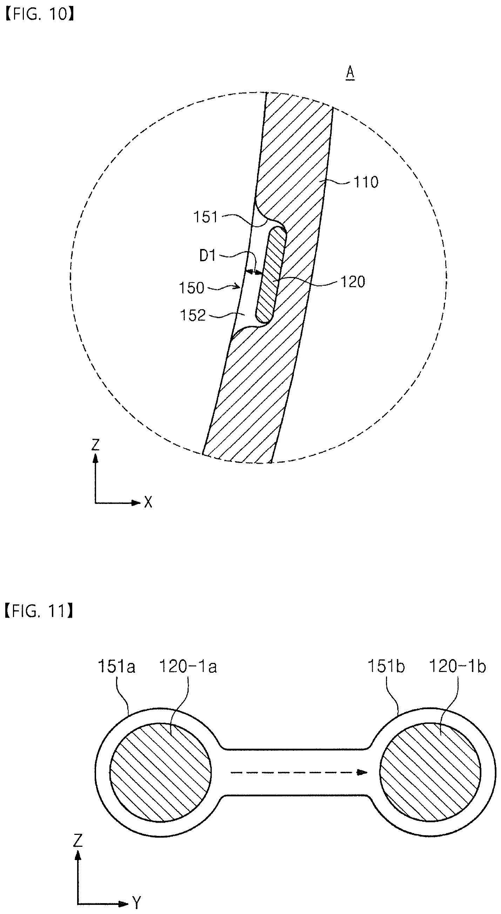

FIGS. 10 to 13 are enlarged views of portion 'A' of FIG. 6, showing electrode pads and a water receiving portion constituting an electrical muscle stimulation system according to an embodiment of the present disclosure. The electrical muscle stimulation system according to the embodiments of FIGS. 10 to 13 differs from the previously described embodiments in that a water receiving portion 150 is provided in the main body 110 and/or the electrode pad 120 to enhance the benefits of electrical muscle stimulation (EMS).

When the user's body directly contacts the electrode pad 120, the electrical stimulation generated by the electrode pad 120 is directly transmitted to the user's body, which may act as a factor that suppresses the EMS benefits. That is, in order to allow an EMS current to smoothly flow between the two pairs of electrode pads 120 through water, it is necessary to prevent the user's body from directly contacting the electrode pad 120, or to provide a space or structure capable of accommodating water between the user's body and the electrode pads 120.

By taking this into consideration, the water receiving portion 150 that accommodates water may be provided in the main body 110 and/or the electrode pad 120. The water receiving portion 150 may be provided to introduce moisture for EMS current formation between the user's body and the electrode pads 120, and through this, the EMS current may flow smoothly via a pair of electrode pads 120, thereby inducing a stronger EMS benefit on the user's body.

The water receiving portion 150 may be provided as a water receiving groove provided in the main body 110 and/or the electrode pad 120, or as a water receiving member (e.g., sponge) provided in the electrode pad 120. In the embodiment of FIG. 10, the water receiving portion 150 may be formed as a water receiving groove 151 provided in the main body 110 corresponding to the bathtub. The water receiving groove 151 may be provided by being recessed from an inner surface 110a of the bathtub corresponding to the main body 110 to form the electrode pad 120. That is, the electrode pad 120 may be installed in a buried manner by being recessed from the inner surface 110a of the main body 110, and accordingly, the water receiving groove 151 may be provided between the inner surface 110a of the main body 110 and the electrode pad 120.

Water in the bathtub may be introduced into the water receiving groove 151. Accordingly, the user's body does not directly touch the electrode pad 120, and an electrical stimulation signal generated by the electrode pad 120 installed in a buried manner in the water receiving groove 151 flows smoothly to corresponding electrode pads (a pair of electrode pads) via water introduced into a water receiving space 152 of the water receiving groove 151, enabling the user to experience an EMS benefit with an appropriate intensity through the EMS current.

The water receiving groove 151 may be formed with a depth D1 that prevents the user's body from directly contacting the electrode pad 120 when the user sits in the bathtub, and may be formed with a depth of, for example, 3 mm or more. Since an excessive size in the depth D1 of the water receiving groove 151 may prevent the EMS current from flowing smoothly to the pair of electrode pads 120, it may be preferable that the water receiving groove 151 be formed with a depth of approximately 1 cm or less.

To allow the EMS current to flow smoothly via the pair of electrode pads 120 received in the pair of water receiving grooves 151, each water receiving groove 151 may be provided with a shape in which the width of the water receiving groove 151 gradually increases from the bottom surface (bottom surface) of the water receiving groove 151 where the electrode pad 120 is placed (the lower area of the water receiving groove) toward the surface of the bathtub corresponding to the main body 110 (the upper area of the water receiving groove).

Meanwhile, an absorbent material (such as a conductive sponge or a grid-shaped conductive silicon material) having electrical conductivity may be additionally provided in the water receiving space 152 within the water receiving groove 151. In this case, the EMS current may flow more smoothly through the conductive absorbent material provided in the water receiving space and moisture introduced into the absorbent material, thereby allowing the user to experience the EMS benefit more effectively.

Referring to FIG. 11, a water receiving long groove 153 may be additionally provided between a pair of water receiving grooves 151a that respectively receive a pair of electrode pads 120-1a and 120-1b. According to the embodiment of FIG. 11, an EMS current may flow smoothly between the pair of electrode pads 120-1a and 120-1b via water introduced into the water receiving long groove 153 provided between the pair of water receiving grooves 151a.

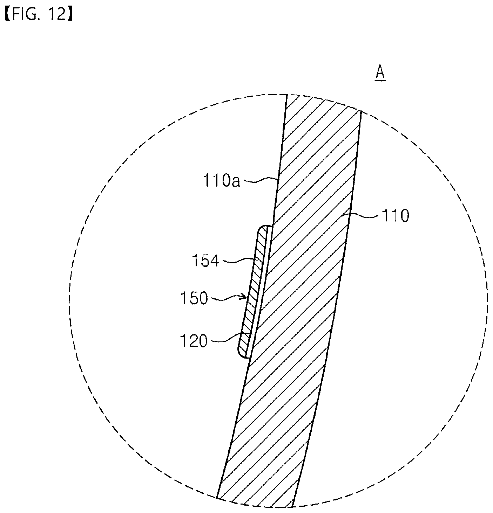

Referring to FIG. 12, the water receiving portion 150 may include a water receiving pad 154 provided on the electrode pad 120. The water receiving pad 154 may serve to separate a user's body from the electrode pad 120 and contain water such that an electrical signal generated by the electrode pad 120 is able to flow smoothly to the pair of electrode pads via the water.

The water receiving pad 154 may be made of a material capable of absorbing water, such as a conductive or non-conductive sponge, or a conductive silicone material containing a conductive material such as carbon black. The water receiving pad 154 may relieve the discomfort felt when a user's body directly contacts the electrode pad 120 or the hole of the water receiving groove when the user sits in a bathtub.

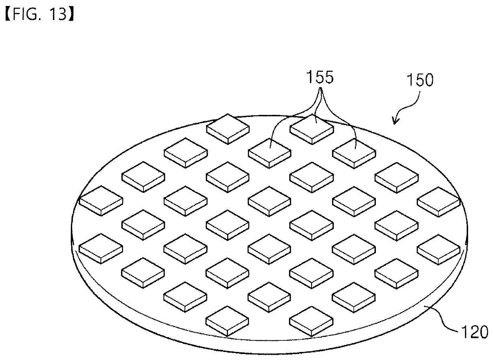

Referring to FIG. 13, the water receiving portion 150 may include a water receiving groove 155 patterned on the upper surface of the electrode pad 120. An electrical pulse is effectively transmitted between the pair of electrode pads 120 via water introduced into the water receiving groove 155 patterned on the upper surface thus, the user may effectively experience the EMS benefit.

As described above, according to the embodiment of the present disclosure, it is possible to implement rolling massage benefit that provides a massage while moving along the body parts of the user through individual channel control and to provide massage continuously/sequentially along the body parts of the user by controlling an electrical stimulation pattern applied through multiple pairs of electrode pads.

In addition, according to the embodiment of the present disclosure, it is possible to provide a water receiving portion in an electrode pad to effectively induce electrical stimulation to a user through water received in the water receiving portion, and to control reinforcement/cancellation interference of electrical signals between adjacent electrode pads to induce appropriate electrical stimulation required by the user.

Meanwhile, the electrical muscle stimulation system according to the embodiment of the present disclosure may select a plurality of operating electrode pads 120 to generate an electrical signal according to a target position and target intensity of electrical muscle stimulation from among a plurality of electrode pads. The control unit may differently determine a plurality of control signals for each of a plurality of setting modes set in advance according to a type of user (gender, age group, body type, or the like), a body part in which electrical muscle stimulation is to be induced and the type of electrical muscle stimulation (exercise, rehabilitation, fatigue recovery, massage, or the like). The plurality of setting modes may be set for various body parts, such as, chest, shoulders, back, waist, thighs, and arms.

The control unit may determine a control signal to be applied to an operating electrode pad selected from among the plurality of electrode pads 120, and transmit the control signal to the operating electrode pad via an individual channel. Accordingly, the control signal may be independently generated for each of channels respectively corresponding to a plurality of setting modes, and the frequency and intensity of an electrical signal output through the plurality of operating electrode pads may be adjusted.

The control unit may select one or more operating electrode pads from among the plurality of electrode pads according to a target position and a target intensity for electrical muscle stimulation to be induced in the user's human body. The control unit may determine the frequency and intensity of an electrical signal according to the position, target position, and target intensity of the plurality of operating electrode pads. The control unit may set an electrical muscle stimulation area within a set distance centered on the target position according to the target position and target intensity for electrical muscle stimulation. The electrical muscle stimulation area may be set to, for example, a circle, a sphere, a square, or the like. The control unit may select electrode pads located within the electrical muscle stimulation area among the plurality of electrode pads as the operating electrode pads.

The control unit may determine the stimulation intensity and frequency of the control signal to be applied to the operating electrode pads according to the target position and target intensity of the electrical muscle stimulation. An electrical pulse corresponding to the target intensity (target magnitude) is induced at the target position for electrical muscle stimulation, and the user may focus the benefits of exercise, rehabilitation, massage, or the like on a specific body part desired by the user.

The electrical muscle stimulation system and the electrical muscle stimulation method according to the described embodiment of the present disclosure may be implemented in the form of a program which a processor is able to execute. Here, the program may include program instructions, data files, data structures, and the like, alone or in combination. The program may be designed and manufactured using a machine language code or a higher level code.

The program may be specially designed to implement the aforementioned method and may be implemented using functions or definitions well-known and available to those skilled in the computer software arts. The program for implementing the above-described method may be recorded on a recording medium readable by a processor. The recording medium may be a memory.

The memory may store a program for performing the above-described operation and an operation to be described below, and the memory may execute the stored program. When a processor and a memory are provided in plural, the processor and the memory may be integrated in one chip, or may be provided in physically separate positions. The memory may include a volatile memory, such as a static random access memory (S-RAM) or a dynamic random access memory (D-RAM) for temporarily storing data.

In addition, the memory includes a non-volatile memory, such as a Read Only Memory (ROM), an Erasable Programmable Read Only Memory (EPROM), and an electrically Erasable Programmable Read Only Memory (EEPROM), for long-term storage of control programs and control data. The processor may include various logic circuits and arithmetic circuits, process data according to a program provided from the memory, and generate a control signal according to a processing result.

At least one component may be added or deleted in response to the performance of the components described above. In addition, it will be readily understood by those of ordinary skill in the art that the mutual positions of the components may be changed in response to the performance or structure of the system.

The embodiments of the disclosure have been described above with reference to the accompanying drawings. A person skilled in the art to which this disclosure pertains will understand that the present disclosure may be practiced in forms different from the disclosed embodiments without changing the technical idea or essential features of the present disclosure. The disclosed embodiments are illustrative and should not be construed as limiting.

According to the embodiment of the present disclosure, it is possible to provide an electrical muscle stimulation system and an electrical muscle stimulation method capable of implementing a rolling massage benefit that provides a massage while moving along body parts of a user by sequentially applying an electrical stimulation signal to a plurality of electrode pads through individual channel control.

In addition, according to the embodiment of the present disclosure, it is possible to provide a massage continuously/sequentially along body parts of a user by controlling an electrical stimulation pattern applied through a plurality of electrode pads.

In addition, according to the embodiment of the present disclosure, it is possible to provide a water receiving portion in an electrode pad to effectively induce an electrical stimulation to a user through water received in a water receiving portion.

In addition, according to the embodiment of the present disclosure, it is possible to control the reinforcement/cancellation interference of electrical signals between adjacent electrode pads such that an appropriate electrical stimulation required by a user is induced.

The benefits obtainable from the present disclosure are not limited to the benefits mentioned above. Other benefits not mentioned will be clearly understood by those of ordinary skill in the art to which the present disclosure pertains from the following description.

While the present disclosure has been described with reference to embodiments thereof, it will be apparent to those of ordinary skill in the art that various changes and modifications may be made thereto without departing from the spirit and scope of the present disclosure as set forth in the following claims.

## Claims

1. An electrical muscle stimulation system comprising:
a main body provided to be wearable by a user, or provided in a bathtub-like form or in a form capable of coming into contact with a user's body;
a plurality of electrode pads provided on the main body and spaced apart from each other at positions corresponding to various parts of the user's body; and
a control unit configured to individually control operations of the plurality of electrode pads by outputting control signals to the plurality of electrode pads through individual channels,
wherein the control unit is configured to sequentially apply an electrical stimulation signal according to an arrangement order of the plurality of electrode pads to implement a rolling massage mode.

2. The electrical muscle stimulation system of claim 1, wherein the electrical stimulation signal includes a stimulation increase interval in which an electrical stimulation gradually increases, a stimulation maintenance interval in which the electrical stimulation is maintained, and a stimulation decrease interval in which the electrical stimulation gradually decreases.

3. The electrical muscle stimulation system of claim 1 or 2, wherein electrical stimulation signals generated from adjacent pairs of electrode pads include an overlapping stimulation interval that partially overlaps each other, and
wherein, in the overlapping stimulation interval, electrical stimulation signals generated from one pair of electrode pads gradually decrease, and electrical stimulation signals generated from the other pair of electrode pads gradually increase.

4. The electrical muscle stimulation system of claim 3, wherein the overlapping stimulation interval includes:
a resonance prevention interval in which electrical stimulation signals generated from two pairs of electrode pads are set such that the a current generated from the one pair of electrode pads and a current generated from the other pair of electrode pads do not cause resonance; and
a resonance induction interval in which the electrical stimulation signals generated from the two pairs of electrode pads are set such that the current generated from the one pair of electrode pads and the current generated from the other pair of electrode pads cause resonance.

5. The electrical muscle stimulation system of any of the preceding claims, further comprising:
a water receiving portion provided in at least one of the main body and the electrode pad to receive water between the user's body and the electrode pad.

6. The electrical muscle stimulation system of claim 5, wherein the water receiving portion includes a water receiving groove including a water receiving space provided in the main body to receive water, and the electrode pad includes a recess electrode installed in a buried manner within the water receiving groove.

7. The electrical muscle stimulation system of claim 5 or 6, wherein the water receiving portion includes a water receiving member provided on the electrode pad or a water receiving groove patterned an upper surface of the electrode pad.

8. An electrical muscle stimulation method comprising: individually controlling, by a control unit, operations of a plurality of electrode pads provided in a main body by outputting control signals to the plurality of electrode pads through individual channels, the main body being provided to be wearable by a user, or provided in a bathtub-like form or in a form capable of coming into contact with a user's body, and the plurality of electrode pads being provided on the main body and spaced apart from each other at positions corresponding to various parts of the user's body,
wherein the controlling includes sequentially applying an electrical stimulation signal according to an arrangement order of the plurality of electrode pads to implement a rolling massage mode.

9. The electrical muscle stimulation method of claim 8, wherein the electrical stimulation signal includes a stimulation increase interval in which electrical stimulation gradually increases, a stimulation maintenance interval in which electrical stimulation is maintained, and a stimulation decrease interval in which electrical stimulation gradually decreases.

10. The electrical muscle stimulation method of claim 8 or 9, wherein electrical stimulation signals generated from adjacent pairs of electrode pads include an overlapping stimulation interval that partially overlaps each other, and
wherein, in the overlapping stimulation interval, electrical stimulation signals generated from one pair of electrode pads gradually decrease, and electrical stimulation signals generated from the other pair of electrode pads gradually increase.

11. The electrical muscle stimulation method of claim 10, wherein the overlapping stimulation interval includes:
a resonance prevention interval in which electrical stimulation signals generated from two pairs of electrode pads are set such that the a current generated from the one pair of electrode pads and a current generated from the other pair of electrode pads do not cause resonance; and
a resonance induction interval in which the electrical stimulation signals generated from the two pairs of electrode pads are set such that the current generated from the one pair of electrode pads and the current generated from the other pair of electrode pads cause resonance.

12. A non-transitory recording medium storing a computer program which, when readable by a computer, executes the electrical muscle stimulation method any claims 8 to 11.
